(19) Europäisches Patentamt
European Patent Office
Office européen des brevets

(11) Publication number: **0 001 019**
**A1**

# (12) EUROPEAN PATENT APPLICATION

(21) Application number: **78300327.0**

(22) Date of filing: **29.08.78**

(51) Int. Cl.²: **C 07 C 109/14,** A 01 N 9/20

(30) Priority: **29.08.77 AU 1437/77**

(43) Date of publication of application: **07.03.79**
**Bulletin 79/5**

(84) Designated Contracting States: **CH DE GB**

(71) Applicant: **COMMONWEALTH SCIENTIFIC AND INDUSTRIAL RESEARCH ORGANISATION, Limestone Avenue, Campbell Australian Capital Territory (AU)**

(72) Inventor: **Holan, George, 86 Were Street, Brighton Victoria (AU)**

(74) Representative: **Lawrence, Peter Robin Broughton et al, Gill, Jennings & Every 53-64 Chancery Lane, London WC2A 1HN (GB)**

## (54) Phenylhydrazones, their use in insecticidal compositions and methods for their preparation.

(57) Compounds of the general formula

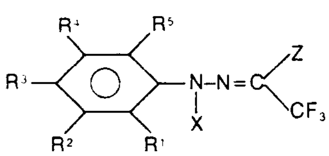

wherein $R^1$, $R^2$, $R^3$, $R^4$ and $R^5$ are each independently H, Cl, Br, $NO_2$ or $CF_3$; X = H or Br; and Z is H, $CH_3$ or $CF_3$.

The compounds are all potent inhibitors of oxidative phosphorylation in rat liver mitochondria and have other biological activity, for example, insecticidal activity.

The compounds are made (A) where X = H and Z = $CF_3$, by reacting the appropriately substituted phenylhydrazine with hexafluoroacetone in a basic organic solvent in the presence of an acidic dehydrating agent; (b) where X = H and Z is H or $CH_3$ by reaction of the phenylhydrazine with 1,1,1-trifluoroacetone or trifluoroacetaldehyde in a suitable solvent in the presence of an acid catalyst; (c) where X = Br can be made from the corresponding compound where X = H by direct bromination.

The invention includes insecticidal compositions containing the compounds.

EP 0 001 019 A1

0001019

TITLE MODIFIED
see front page



C.S.I.R.O.
GJE678/339

# "BIOLOGICALLY ACTIVE HYDRAZONES"

This invention relates to new biologically active hydrazones and especially to those having insecticidal activity. The invention also provides methods for preparing these compounds and to new insecticidal compositions containing the compounds.

Throughout this specification, where the contextpermits, the word "insect" is used in its broad common usage and includes spiders, mites, nematodes and other pests which are not classed as insects in the strict biological sense. Thus the term implies reference not only to those small invertebrate animals belonging mostly to the class Insecta, comprising six-legged, usually winged forms, such as beetles, bugs, flies and the like, but also to other allied classes of arthropods whose members are wingless and usually have more than six legs, such as spiders, wood lice and the like, and especially to the order Acaridae which includes the mites and ticks. The words "insecticide" and "insecticidal" are similarly used.

The compounds provided by this invention have the general formula I

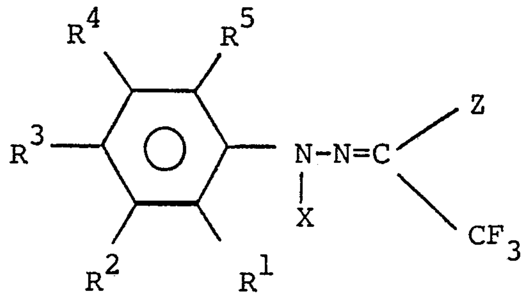

wherein $R^1$, $R^2$, $R^3$, $R^4$ and $R^5$ are each independently H, Cl, Br, $NO_2$ or $CF_3$; X = H or Br; and Z is H, $CH_3$ or $CF_3$ .

These compounds are all potent inhibitors of oxidative phosphorylation in rat liver mitochondria and have other biological activity. For example, the compound with $R^1=R^3=NO_2$, $R^2=R^4=R^5=H$, X=H, and $Z=CF_3$ shows insecticidal properties against housefly (both susceptible and resistant strains), Australian sheep blowfly, and mosquitos. It is also acaricidally active against the spider mites Tetranychus urticae and Tetranychus ludeni, and the larvae of the Queensland cattle tick Boophilus microplus. The compound is also active against ants and is a contact repellent for flies. The N-bromo compounds, where X = Br, have equal or enhanced insecticidal activity in some species when compared to the corresponding compound where X = H. The reason for the enhanced activity appears to be the greater ease of penetration of the N-bromo compound through the cuticle of the insect.

The invention includes not only insecticidal compounds, and the insecticidal use of compounds, of formula 1 but also the novel compounds themselves. Preferred novel compounds of the invention are compounds of formula 1 but subject to the proviso that when Z is $CH_3$ and X, $R^2$, $R^4$ and $R^5$ are all H then $R^1$ and $R^3$ are not both $NO_2$.

Preferred compounds are those wherein $R^1$, $R^2$, $R^3$, $R^4$ and $R^5$ are selected from H, $CF_3$ and $NO_2$,

preferably wherein $R^1$ and $R^3$ are individually selected from H, $CF_3$ and $NO_2$ (preferably $CF_3$ and $NO_2$), $R^2$ and $R^4$ are individually selected from $CF_3$ and H (preferably H) and $R^5$ is $NO_2$ or H (preferably H).

Details of the biological activity of compounds of the invention are given in the Examples.

These compounds are thus useful as insecticides for the control of a wide range of insect pests.

The specific compound mentioned above (the 2:4-dinitrophenylhydrazone of hexafluoroacetone) was claimed to have been made by R.N. Haszeldine in a paper titled "Addition of free radicals to unsaturated systems"

(J. Chem. Soc., 1953, 3565). In that paper there is a mention of the comparison of the spectrum of a "known specimen" of this derivative and of the spectrum of the semicarbazone of hexafluoroacetone with that of a "known spectrum". No mention is made of the preparation of either, or of a previous reference or of the source of the "known specimens".

On the other hand, in the review of hexafluoroacetone in Fluorine Chemistry Review, Vol. 1, Ed. P. Tarrant, published Marcel Dekker Inc., New York 1967, the authors G.C. Krespan and W.J. Middleton state on page 155

> "Conditions for preferential elimination of water from other types of $-NH_2$ adducts of hexafluoroacetone have not been found..... A good example is the preparation of hexafluoroacetone semicarbazone. As first reported by several workers it was apparently obtained as the hydrate - that is, as the adduct of semicarbazide with hexafluoroacetone. Not until Zeifman et. al. reacted semicarbazide with hexafluoroacetone N-phenylimine was an adduct obtained which could be dissociated to a true semicarbazone".

There is no mention of a substituted hydrazone in this review.

We have assumed that in the absence of any stated method of preparation of hexafluoroacetone 2:4-dinitrophenylhydrazone, the method attempted by Haszeldine would have been the standard reaction of 2,4-dinitrophenylhydrazine with the ketone or its hydrate in a solvent using conventional acid catalysis. Our attempts to use this method of preparation failed; after refluxing either hexafluoroacetone or its mono or sesquihydrate in ethanol, dimethoxyethane or heating in dimethyl sulphoxide, with 2,4-dinitrophenylhydrazine or its acid salt for periods ranging from 30 min. to 8 days, no hydrazone could be isolated. A precipitate

which formed in three instances when an excess of sulphuric acid was used as the condensing agent was isolated and proved to be 2,4-dinitrophenylhydrazine sulphate. In two experiments involving prolonged reflux in the presence of excess acid 2,4-dinitroaniline was isolated as the sole product of reaction. These products were identified by infrared spectra and mixed melting points with authentic samples.

The only product identified as the phenylhydrazone was prepared by the method described in the present specification. The elemental analysis results, the infrared spectrum, mass spectrum and proton magnetic resonance spectrum of this product were all consistent with the correct structure. We conclude therefore that Haszeldine did not in fact make hexafluoroacetone 2:4-dinitrophenylhydrazone.

The 2:4-dinitrophenylhydrazone of 1,1,1-trifluoroacetone (which, unlike the hexafluoroacetone derivative, can be made by conventional methods) has also been reported (A.L. Henne, M.S. Newman, L.L. Quill and R.A. Stoniforth, J. Amer. Chem. Soc. 69, 1819 (1947) and A. Sykes, J.C. Tatlow and C.R. Thomas, J. Chem. Soc. (1956), 853) but no biological activity has been ascribed to it.

The invention also provides methods for preparing the compounds of formula I.

In the case where X=H and $Z=CF_3$ in formula I, the compounds are prepared by reacting the appropriately substituted phenylhydrazine (II;$R^1$ to $R^5$ as defined above) with hexafluoroacetone (III;$Z=CF_3$) in a basic organic solvent (for example pyridine) in the presence of an acidic dehydrating agent (such as phosphorus oxychloride or thionyl chloride).

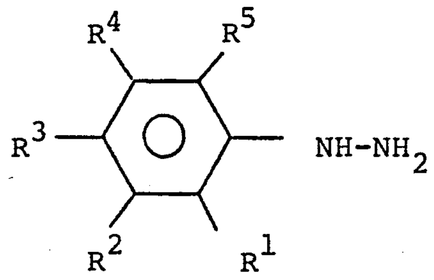

II

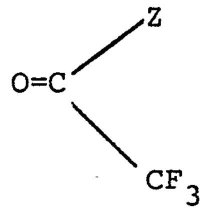

III

It is preferred that the dehydrating agent (which also has a catalytic action) should be present in an approximately equimolar (or greater) amount with respect to the phenylhydrazine.

Where X=H and Z in formula I is H or $CH_3$, the hydrazones can be made by the conventional method, i.e. reaction of the phenylhydrazine (II) with 1,1,1-trifluoroacetone (III, $Z=CH_3$) or trifluoroacetaldehyde (III, Z=H) in a suitable solvent (e.g. ethanol) in the presence of an acid catalyst (e.g. sulphuric acid).

The compounds where X=Br can be made from the corresponding compound where X=H by direct bromination, preferably by reaction with bromine in glacial acetic acid buffered with sodium acetate.

The compounds described herein may be formulated as insecticidal compositions with suitable solid and or liquid carriers either as working compositions having a concentration below 5% by weight or as concentrates having a concentration of 5 to 95% by weight. Thus they may be incorporated in a suitable inert solvent, or mixture of solvents, or in a solid mixture, with or without other substances, such as wetting, dispersing and sticking agents. The compounds may be employed in such compositions either as the sole toxic agent or in combination with other insecticides such as pyrethrum, rotenone, or with fungicidal or bactericidal agents, to provide compositions useful for household and agricult ural dusts and sprays, textile coating and impregnation,

and the like. The compounds may be dissolved in suitable organic solvents to provide solutions of enhanced utility. The new compounds may also be placed in aqueous suspension by dispersing organic solvent solutions of the compounds in water. The new compounds may also be mixed with an inert, finely divided, solid diluent or carrier. The insecticidal compounds may be admixed in their original forms or in solution.

The preparation and properties of the compounds of the invention are illustrated by the following specific examples. It should be noted, of course, that these examples are intended to be illustrative of the methods and procedures utilized in preparing the compounds and that they are not intended to be restrictive or to be regarded as embodying the only way in which the compounds can be formed and recovered.

All temperatures are in Celsius.

(A) Preparation of 3,5-Bis-(trifluoromethyl)phenylhydrazine hydrochloride

3,5-Bis-(trifluoromethyl)aniline (6.7g) was diazotised with $NaNO_2$ (2.07g) in hydrochloric acid (conc. 20 ml). Insoluble material was filtered off. The clear solution was cooled to $-10^{o}$ and $SnCl_2.2H_2O$ (27 g) added dropwise. After standing overnight the solution was filtered and the orange precipitate was sublimed. Yield of the hydrochloride was 5.7 g (67.7%).

(B) Preparation of 4-Nitro,2-trifluoromethylphenylhydrazine hydrochloride

Hydrazine hydrate (16.45 g) in ethanol (110 ml), was added to 2-chloro,3-nitrobenzotrifluoride (22.5 g) and the solution refluxed for 22 h. After quenching in water the solution was extracted with methylene chloride, and the oil obtained after evaporation was dissolved in ether and saturated with hydrogen chloride. The hydrochloride of the phenylhydrazine was filtered off and the product (7.4 g) characterized as the hydrazone derivative of acetone (m.p. $122^{o}$).

(C) General Method for the Preparation of Phenylhydrazones of Hexafluoroacetone

The appropriate phenylhydrazine (0.01 mole) and hexafluoroacetone (anhydrous, trihydrate or sesquihydrate; 0.012 mole) in pyridine (10 ml) were cooled to $0^{o}$ and the acidic dehydrating agent (e.g. phosphorus oxychloride or thionyl chloride) added gradually. After the strong exothermic reaction had ceased the mixture was heated on the water bath for 20 min. After cooling, the solidified mixture was decomposed with water and extracted with a solvent (ether, chloroform, benzene or methylene chloride). The solvent extract was washed with acid, water, and dried. After evaporation, the solid derivatives were purified by recrystallization; where oils were obtained they were purified by chromatography on silica gel using chloroform as an eluent.

(D) General Method for the Preparation of Phenylhydrazones of 1,1,1-trifluoroacetone

The appropriate phenylhydrazine (0.01 mole) was dissolved in ethanol (25 ml) by the addition of conc. sulfuric acid (0.5 ml). 1,1,1-Trifluoroacetone (0.012 mole) was then added and the precipitate formed filtered off after standing for 2 h. If no precipitate appeared the solution was refluxed for 2 h. The products were recrystallised from ethanol.

(E) General Method for the N-Bromination of Hydrazones

The appropriate hydrazone (0.003 mole) is dissolved in 20 ml of glacial acetic acid. Bromine (0.0063 mole) and sodium acetate (0.0122 mole) are added and the mixture is left standing at room temperature for 72 hours. It is then heated to $50^{o}C$ for 2 hours and evaporated to dryness at $80^{o}C$ under vacuum. The residue is quenched with 50 ml of distilled water, the insoluble material is separated and dried at $50^{o}C$ under vacuum then recrystallized from petroleum spirit - carbon tetrachloride mixture or distilled at high vacuum.

EXAMPLES 1 to 11

Using the general methods described above, the compounds listed in Table I were produced from the appropriately substituted starting materials in the yields indicated. Melting or boiling points and elemental analysis are given. The structures of the products were confirmed by I.R. and N.M.R. spectra.

TABLE I

EXAMPLES OF BIOLOGICALLY ACTIVE HYDRAZONES

| Ex. No. | Formula I | | | | | | | M.p./B.p. | Elemental Analysis Calculated/Found | | | | Yield % |
|---|---|---|---|---|---|---|---|---|---|---|---|---|---|
| | $R^1$ | $R^2$ | $R^3$ | $R^4$ | $R^5$ | X | Z | | C | H | N | F | |
| 1 | H | H | H | H | H | H | $CF_3$ | 95° @ 15mm | 42.21/41.99 | 2.36/2.37 | 10.9/10.8 | 44.5/44.2 | 32 |
| 2 | H | $CF_3$ | H | $CF_3$ | H | H | $CF_3$ | 88° @ 2.0mm | 33.69/33.85 | 1.03/1.09 | 7.2/7.2 | 58.1/57.9 | 40 |
| 3 | $NO_2$ | H | $NO_2$ | H | H | H | $CF_3$ | 89° | 31.23/31.25 | 1.17/1.19 | 16.2/16.1 | 32.9/33.2 | 53 |
| 4 | $CF_3$ | H | $NO_2$ | H | H | H | $CF_3$ | 58° | 32.53/32.63 | 1.09/1.21 | 11.4/11.3 | 46.3/46.3 | 18 |
| 5 | $NO_2$ | H | $CF_3$ | H | H | H | $CF_3$ | 56° | 32.53/32.42 | 1.09/1.08 | 11.4/11.2 | 46.3/45.9 | 75 |
| 6 | $NO_2$ | H | $NO_2$ | H | H | H | $CH_3$ | 139° | 37.00/37.28 | 2.42/2.50 | 19.2/19.3 | 19.5/19.6 | 71 |
| 7 | $NO_2$ | H | $CF_3$ | H | $NO_2$ | H | $CH_3$ | 169° | 33.35/33.60 | 1.68/1.77 | 15.6/15.3 | 31.7/31.7 | 93 |
| 8 | $NO_2$ | H | $CF_3$ | H | $NO_2$ | H | H | 125° | 31.23/31.21 | 1.17/1.22 | 16.2/16.2 | 32.9/33.1 | 75 |
| 9 | $NO_2$ | H | $NO_2$ | H | H | Br | $CF_3$ | 55° | 25.41/25.69 | 0.71/0.93 | 13.2/13.2 | 26.8/26.9 | 33 |
| 10 | $CF_3$ | H | $NO_2$ | H | H | Br | $CF_3$ | 85° @ $10^{-5}$ mm | 26.81/26.73 | 0.67/0.91 | 9.4/9.6 | 38.2/38.0 | 14 |
| 11 | $NO_2$ | H | $CF_3$ | H | H | Br | $CF_3$ | 83° | 26.81/26.93 | 0.67/0.73 | 9.4/9.6 | 38.2/38.1 | 63 |

(F) Activity Test Methods

The biological activity of the new hydrazones was examined in a series of tests, the results of which are collected in Table II.

Insecticidal activity and repellency were investigated against the common housefly, Musca domestica, and the sheep blowfly, Lucilia cuprina. Acaricidal activity was measured against larvae of the Australian cattle tick, Boophilus microplus and against the Tetranychid mites. They were also tested against ants and the yellow fever mosquito Aedes aegypti. The methods used were as follows:-

(i) Housefly

(a) Insecticidal Activity

Tests were carried out using a standard DDT-susceptible strain (WHO/IN/1) of M. domestica. The compound was applied in an acetone solution by micro-syringe to the dorsum of the thorax of two day old female flies reared from pupae of average weight 2.2 - 2.5 gm/100 pupae. The adult flies were fed on water and sugar-only diet and maintained at 26°C and 70% RH. The mortalities were counted at 48 hours after treatment and compared with acetone-treated controls. Flies unable to move or stand normally were considered dead. The $LD_{50}$ value was obtained from a logit computer programme based on three replicates of 10 flies at each dose level. The $LD_{50}$ value for DDT determined under the same conditions was 0.26 μg/fly.

(b) Insect Repellency

Repellency tests were carried out on the same strain of housefly as in the mortality tests. Female flies at least two days old, not previously fed protein, were taken the day before the test, anaesthetized with $CO_2$ and counted into holding containers of twenty flies each. These were supplied with water and solid sucrose. On the day of the tests the food and water were removed in the morning (0900 hr). As the tests were performed only between 1200 hr and 1730 hr, the flies were

therefore starved for a minimum of three hours before testing.

The test involved the use of attractant baits to which the candidate compound was applied. These were exposed to the flies and the number of flies landing on each bait counted. The baits consisted of aluminium caps of area $5.94cm^2$ filled with bakers yeast mixed with water and slightly heated to form a solid surface film.

Eight lots of 20 flies were used in a run in which seven discs were treated with a graded dilution series of the test chemical using acetone as solvent, together with one disc treated with acetone as a control. The concentrations of the compound ranged from 0.031 μg/μl doubling at each level up to 2.0 μg/μl. One hundred microlitres of each solution was pipetted evenly over the surface of each disc and left until the acetone had evaporated.

The flies to be used were released into standard 205 mm x 205 mm x 255 mm mesh cages and left to acclimatize in the test room maintained at a temperature of $26^oC \pm 1^oC$ and humidity approximately 60%, for ten minutes before introducing the treated discs into each cage. Before use the discs were marked on the reverse sides and then randomly mixed to avoid bias in counting. In the thirty minute period of the test the number of flies on the surface of each disc was counted in the first and second minute after introducing the baits and thereafter every two minutes. In this way sixteen counts were obtained for each concentration, the totals of which were then used for a regression analysis of the concentration effect. Also a total number of landings for each concentration was obtained and used for calculation of the Index of Repellency (IR). All replicate tests were carried out with fresh flies and baits, and the compounds were tested in three replicate runs.

The total number of flies counted on each disc for the seven concentration levels was summed and averaged. In the following formula this figure is designated (N), where (C) equals the number of flies counted on the control:-

$$\frac{C - N}{C + N} \times 100 = \text{Index of Repellency (IR)}$$

(ii) Sheep Blowfly

(a) Insecticidal Activity

The compounds were tested for activity against a dieldrin susceptible strain (LBB) which had been collected before dieldrin usage in the field.

The test compound was applied in acetone solution, 0.5 µl dispensed with a Drummond micropipette to the dorsum of the thorax of 2-3 day old females. Adult flies were fed on water and sugar only and maintained at 25°C and 60-70% RH. The mortalities were determined after 24 hours. Moribund flies were regarded as dead. The LD 50 values, in terms of concentration, were interpolated from a probit/log dose graph using a computer program and are converted to µg in Table II.

Comparative $LD_{50}$ figures for DDT and dieldrin are 0.17 and 0.025 µg/insect.

(b) Repellency

Repellency was determined as described above in the housefly tests, except that the baits consisted of an agar gel containing fresh beef blood.

(iii) Cattle Tick

The determinations of mortalities were carried out in packets on 7 - 14 day old larvae of the cattle tick Boophilus microplus using the method described by B.F. Stone in "Inheritance of resistance to organophosphorus acaricides in the cattle tick Boophilus microplus". Aust. J. Biol. Sci. 21, 309-319,

(1968). Mortalities were counted 24 hours after the application of the compounds.

(iv) Tetranychid mites

The mites, *Tetranychus urticae* and *Tetranychus ludeni* were maintained on dwarf french bean plants. For testing, discs of bean leaf were cut from fresh plants with a 20mm cork borer. Petri dishes were set up with moist cotton wool in each and three discs of leaf on top with the ventral side of the leaf uppermost.

The compounds to be tested were made up in a 1:1 acetone/water mixture together with a drop of Triton X-100, to facilitate mixing and spreading on the leaf surface. A serial dilution was then made using the same solvent mixture.

The solutions were pipetted and spread onto the discs at a rate of 30 µl/disc and the solvent allowed to evaporate before placing mites on the treated surfaces. Adult female mites were removed from infected leaves with a fine bristle brush and placed on the surface, five to each disc. This gave a sample size of 15/concentration. The petri dishes (including a control treated only with solvent) were then transferred to a holding oven and kept in the dark at 26°C.

Mortalities were read at 24 hours (and longer when required) along with counts of the number of mites leaving the treated surfaces.

(v) Ants

Serial dilutions of the compounds to be tested were made up in acetone. Glass petri dishes (9cm diameter) were treated on their inside surfaces by swirling 0.5ml of test solution in each and then allowing the acetone to evaporate. This produced a relatively uniform treated surface on which to place the ants. The sides of the dishes were coated with fluon to prevent the ants walking off the surfaces. An acetone control was also prepared for each test.

The ants (*Pheidole megacephala*) were

collected using a meat bait inside a plastic container. Without anaesthetizing, ants were then shaken onto the treated surfaces. Mortalities were read at 1 hour and again at 3 hours.

(vi) Yellow fever mosquito larvae

A DDT susceptible strain of the mosquito, *Aedes aegypti*, was used. This strain had been maintained in the laboratory for a number of years out of contact with insecticides. Approximately 10-25 first-instar larvae were placed in 10 ml distilled water with 100 μl of solution of the appropriate concentration of the compound in acetone. The lowest concentration, as ppm in the water to produce complete mortality by 48 hours was taken as the $LC_{100}$. No mortality was observed in acetone-only controls.

(vii) Uncoupling of Oxidative phosphorylation

Preparation of rat liver mitochondria

Rat liver mitochondria were prepared by the method described by Hogeboon[1] in a medium containing 0.25 M sucrose; 10 mM tris-HCl (pH 7.40); 0.5 mM EDTA and finally suspended in the same medium plus bovine-serum albumin (2 mg/ml). Oxidative phosphorylation was measured polarographically at 30°, as described by Estabrook, R.W. et al[2]. The incubation medium contained 0.25 M sucrose; 10 mM tris-HCl (pH 7.4); 5 mM $K_2HPO_4$ (pH 7.4); 0.5 mM EDTA and bovine-serum albumin (2 mg/ml). DNP was added and control tests were carried out using either pyruvate or succinate as substrates.

State 3 and state 4 respiration rates were determined by the method of Chance, B. et al[3]. Percentage uncoupling in the presence of antimetabolites was calculated from the equation

$$100 \times \frac{\text{Rate in the presence of antimetabolite-State 4 rate}}{\text{State 3 rate - State 4 rate}}$$

Relative uncoupling is compared as the concentrations

required to give 50% effects ($I_{50}$).

REFERENCES

1. Hogeboon, G.H. (1955). Fractionation of cell components of animal tissue. Methods of Enzymology (Colowick, S.P. and Kaplan, N.O. editors, Academic Press, New York) 1, 16-22.

2. Estabrook, R.W. (1967). Mitochondrial respiratory control and the polarographic measurement of ADP:O ratios. Methods in Enzymology (Colwick, S.P. and Kaplan, N.O. editors, Academic Press, New York) 10,41-47.

3. Chance, B.T., and Williams, G.R. (1955). Respiratory enzymes in oxidative phosphorylation. J. Biol. Chem. 217, 409 - 427.

EXAMPLE 12

The following are examples of insecticidal or acaricidal compositions in accordance with the invention. All parts are by weight.

(a) Water dispersable powder

The following powdered composition is intended for dispersing in water for application as a spray.

| | |
|---|---|
| Compound of formula I | 50.0 |
| Synthetic fine silica | 30.0 |
| Alkyl aryl sodium sulphonate | 1.5 |
| Methyl cellulose (15 cp.) | .25 |
| Attapulgite | 8.25 |

(b) Spray formulation

The following composition is adapted for spray application.

| | |
|---|---|
| Compound of formula I | 4.0 |
| Pyrethrum | 0.1 |
| Deodorized kerosene | 79.4 |
| Alkylated naphthalene | 16.0 |

(c) Aerosol

The following materials are metered into a suitable 'bomb' container sealed and equipped with a valve in the usual way.

| | |
|---|---|
| Compound of formula I | 3.0 |
| Methylene chloride | 10.0 |
| 'Freon 12' | 43.0 |
| 'Freon 11' | 43.0 |

TABLE II - BIOLOGICAL ACTIVITY OF HYDRAZONES

| Ex. No. | House Fly (WHO/IN/1 strain) | | Australian Sheep Blowfly | | Tetranychus Mites | | Uncoupling of oxidative Phosphorylation (Rat Liver) | Mosquito Larvae (Aedes Spp.) | |
|---|---|---|---|---|---|---|---|---|---|
| | $LD_{50}$ µg/insect | Repell-ency Index | $LD_{50}$ µg/ insect | Repell-ency Index | T.urticae $LD_{50}$ µg/insect | T.Ludenii $LD_{50}$ µg/insect | $I_{50}$ mol/litre | A.australis $LC_{50}$ PPM | A.aegypti $LC_{50}$ PPM |
| 1 | | 51 | | | | 100% @ 32 | | | |
| 2 | | | | | | 0.8 | $2.6 \times 10^{-8}$ | | |
| 3* | .011 | 96 | .022 | 83 | .003 | 0.006 | $5 \times 10^{-8}$ | .08 | .006 |
| 4 | .65 | 82 | 1.1 | 75 | | 0.25 | $1.4 \times 10^{-8}$ | | |
| 5 | 9.3 | | 8.2 | | | - | $6.3 \times 10^{-6}$ | | |
| 6 | > 32 | | | 8 | | 32 | $3.8 \times 10^{-6}$ | | |
| 7 | 5.14 | | 4.36 | 17 | | 0.1 | $6.5 \times 10^{-8}$ | | |
| 8 | | | | | | 0.09 | | | |
| 9 | .02 | 74 | .024 | | | | | | |
| 10 | .73 | | .37 | | | | | | |
| 11 | 5.22 | | 8.21 | | | | | | |

* Compound of Example 3: Cattle tick larvae dipped in 0.1% solution - 100% kill all strains. Brown ants 100% kill at .0019 ppm.

CLAIMS

1. A compound for use as an insecticide characterised in that it has the general formula I

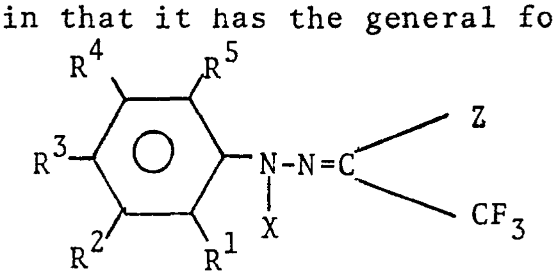

wherein $R^1$, $R^2$, $R^3$, $R^4$ and $R^5$ are each independently H, Cl, Br, $NO_2$ or $CF_3$; X = H or Br; and Z is H, $CH_3$ or $CF_3$.

2. The 2,4-dinitro-phenylhydrazone of 1,1,1-trifluoroacetone for use as an insecticide.

3. A novel compound characterised in that it has the general formula I

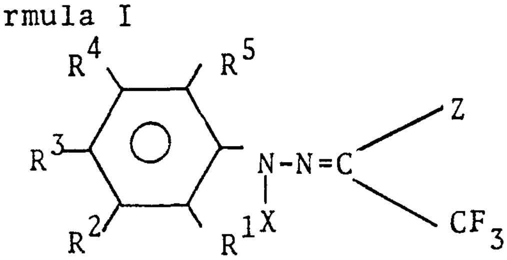

wherein $R^1$, $R^2$, $R^3$, $R^4$ and $R^5$ are each independently H, Cl, Br, $NO_2$ or $CF_3$; X = H or Br; and Z is H, $CH_3$ or $CF_3$ with the proviso that when X, $R^2$, $R^4$, and $R^5$ are H and Z is $CH_3$ then $R^1$ and $R^3$ are not both $NO_2$.

4. The phenylhydrazone and 3,5-bis-(trifluoromethyl)-2,4-dinitro-, 4-nitro-2-trifluoromethyl- and 2-nitro-4-trifluoromethyl-phenylhydrazones of hexafluoroacetone.

5. The 2-nitro-4-trifluoromethyl-phenylhydrazone of 1,1,1-trifluoroacetone.

6. The 2-nitro-4-trifluoromethyl-phenylhydrazone of trifluoroacetaldehyde.

7. The 2,4-dinitro-, 4-nitro-2-trifluoromethyl- and 2-nitro-4-trifluoromethyl-N-bromo-phenylhydrazones of hexafluoroacetone.

8. An insecticidal composition characterised in that it comprises a compound of the general formula I

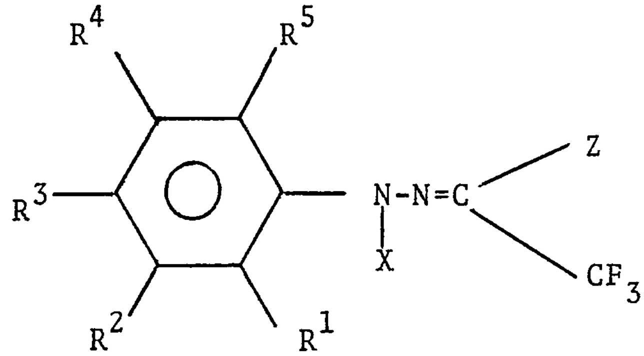

wherein $R^1$, $R^2$, $R^3$, $R^4$ and $R^5$ are each independently H, Cl, Br, $NO_2$ or $CF_3$; X = H or Br; and Z is H, $CH_3$ or $CF_3$, together with a suitable solid or liquid diluent or carrier.

9. A method for preparing compounds of the general formula

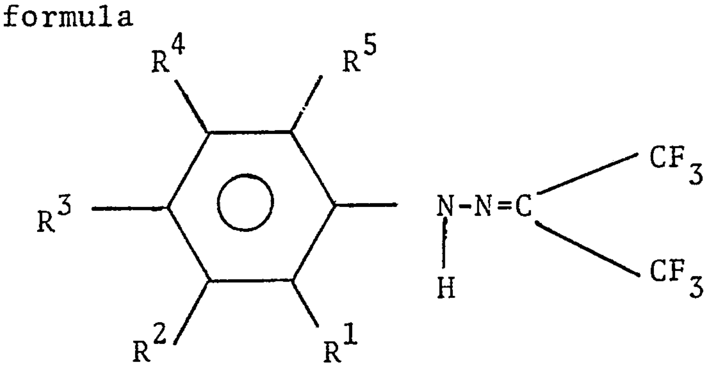

wherein $R^1$, $R^2$, $R^3$, $R^4$ and $R^5$ are each independently H, Cl, Br, $NO_2$ or $CF_3$; characterised in that compounds of formulae IV and V

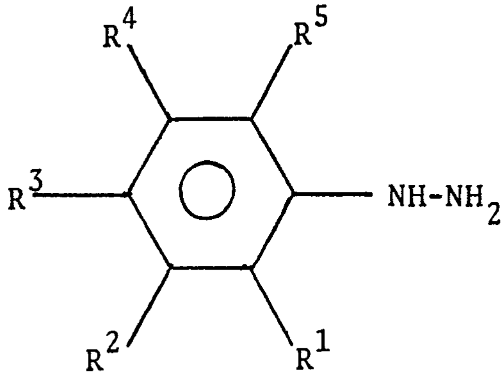 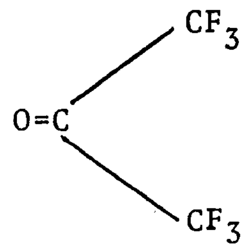

IV V

wherein $R^1$ to $R^5$ are as defined above are reacted in a basic organic solvent in the presence of an acidic dehydrating agent.

10. A method as claimed in claim 9, wherein the basic organic solvent is pyridine and the acidic dehydrating agent is phosphorus oxychloride or thionyl chloride.

11. A method for preparing compounds of the general formula I

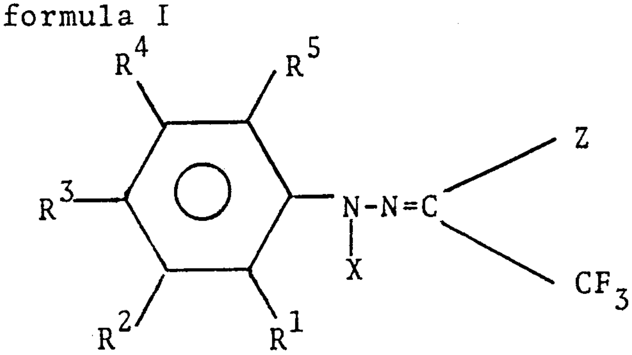

wherein $R^1$, $R^2$, $R^3$, $R^4$ and $R^5$ are each independently H, Cl, Br, $NO_2$ or $CF_3$; X = H and Z is H and $CH_3$ characterised in that compounds of formula IV and VI

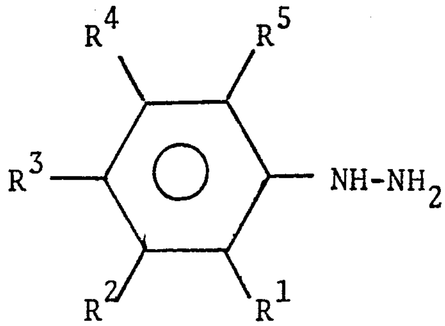

IV

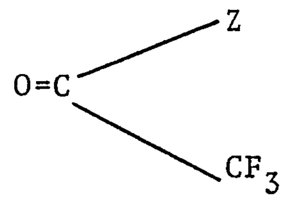

VI

wherein Z is H or $CH_3$ are reacted in a solvent in the presence of an acid catalyst.

12. A method for preparing compounds of the general formula

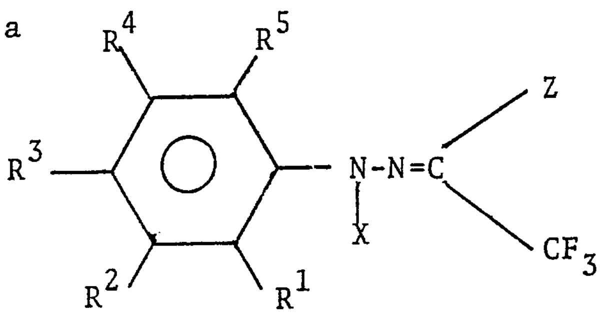

wherein $R^1$, $R^2$, $R^3$, $R^4$ and $R^5$ are each independently H, Cl, Br, $NO_2$ or $CF_3$; X = Br; and Z is H, $CH_3$ or $CF_3$ characterised in that the corresponding compound wherein X is H is brominated.

0001019

European Patent Office

# EUROPEAN SEARCH REPORT

Application number

EP 78 30 0327

## DOCUMENTS CONSIDERED TO BE RELEVANT

| Category | Citation of document with indication, where appropriate, of relevant passages | Relevant to claim |
|---|---|---|
| D,X | JOURNAL OF THE CHEMICAL SOCIETY, 3565-3572 (1953) (cited in the application)<br>* Page 3571, paragraph 2 *<br>-- | 3,7 |
| | US - A - 3 870 505 (G. KAUGARS)<br>* Claim 1 *<br>---- | 1 |

CLASSIFICATION OF THE APPLICATION (Int. Cl.²)

C 07 C 109/14
A 06 N 9/20

TECHNICAL FIELDS SEARCHED (Int.Cl.²)

C 07 C 109/14

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 14-11-1978 | STOOS |